# EUROPÄISCHE PATENTSCHRIFT

(11) **EP 0 431 456 B1**
(45) Veröffentlichungstag und Bekanntmachung des Hinweises auf die Patenterteilung: **15.02.1995**
(21) Anmeldenummer: 90122728.0
(22) Anmeldetag: 28.11.1990
(51) Int. Cl.: C12Q 1/00, G01N 33/48, G01N 33/52

(54) **Verwendung eines schwer löslichen Salzes einer Heteropolysäure zur Bestimmung eines Analyts, entsprechendes Bestimmungsverfahren sowie hierfür geeignetes Mittel**
Use of a heavily soluble salt of a heteropolyacid for the determination of an analyte a corresponding assay and means useful therefor
Utilisation d'un sel soluble lourd d'un hétéropolyacide pour la détérmination d'un composé, essai correspondant et moyen pour cela

(30) Priorität: 02.12.1989 DE 3940010
(43) Veröffentlichungstag der Anmeldung: 12.06.1991
(73) Patentinhaber: BOEHRINGER MANNHEIM GMBH, 68298 Mannheim (DE)
(72) Erfinder: Hönes, Joachim, Dr., W-6144 Zwingenberg (DE); Wielinger, Hans, Dr., W-6940 Weinheim (DE); Unkrig, Volker, Dr., W-6802 Ladenburg (DE)

(56) Entgegenhaltungen:
- EP-A- 0 256 806
- EP-A- 0 262 445
- EP-A- 0 354 441
- F.A. COTTON et al., "Anorganische Chemie", 1967, Verlag Chemie GmbH, Weinheim (DE) und Interscience Publishers, New York, NY (US); Seite 880#
- PHARMACEUTICA ACTA HELVETIAE, Band 64, Nr. 3, März 1989, Bern (CH); P.B. ISSOPOULOS, Seiten 82-85#
- CLINICAL CHEMISTRY, Band 33, Nr. 11, November 1973, Winston-Salem, NC (US); M.L. MATHEKE et al., Seiten 2109-2110#
- CLINICAL CHEMISTRY, Band 12, Nr. 11, November 1966, Winston-Salem, NC (US); B. KLEIN et al., Seiten 816-823#
- CHEMICAL ABSTRACTS, Band 100, Nr. 4, 23 Januar 1984, Columbus, OH (US); C.M. POPA et al., Seite 208, Nr. 26068m#
- INORGANIC CHEMISTRY, Band 14, Nr. 2, Februar 1975, Washington, DC (US); J.J. ALTENAU et al., Seiten 417-421#

## Beschreibung

Die Erfindung betrifft die Verwendung eines schwer löslichen Salzes einer Heteropolysäure zur Bestimmung eines Analyts. Außerdem betrifft die Erfindung ein Verfahren zur Bestimmung eines Analyts mittels Heteropolyblau-Bildung sowie ein hierfür geeignetes Mittel. Schließlich betrifft die Erfindung auch die Verwendung eines schwer löslichen Salzes einer Heteropolysäure zur Herstellung eines Mittels zur Bestimmung eines Analyts durch Heteropolyblau-Bildung.

Heteropolysäuren sind anorganische Polysäuren mit mindestens zwei verschiedenen Zentralatomen. Sie entstehen aus mehrbasischen Sauerstoffsäuren eines Metalls, wie Molybdän, Wolfram, Vanadium und eines Nichtmetalls oder Metalls, wie Phosphor, Silicium, Arsen, Jod, Eisen, Mangan, Kobalt als partielle gemischte Anhydride. Beispiele sind Phosphormolybdänsäure bzw. Phosphorwolframsäure. Heteropolysäuren sind wasserlöslich. Sie sind jedoch nur in saurer Lösung stabil.

Heteropolysäuren des Molybdäns und Wolframs sind seit langem bekannte Reagenzien. Analytische Verwendung finden sie zur Bestimmung von Phosphat und auch zum Nachweis von Arsenat oder Silikat durch Bildung der entsprechenden Heteropolysäuren mit Molybdat oder Wolframat und anschließende Reduktion der Heteropolysäure zu einem blauen Farbstoff, dem sogenannten Heteropolyblau. Die Farbe beruht auf Lichtabsorption durch Elektronenübergang zwischen fünf-und sechswertigem Molybdän oder Wolfram. Das Heteropolyblau aus Heteropolysäuren ist jeweils ein Farbstoff hoher Extinktion, der in Abhängigkeit von der Wellenlänge ein sehr breites Absorptionsmaximum aufweist. Dies ist typisch für Charge-transfer-Absorptionsbanden.

Heteropolysäuren zum Nachweis von reduzierenden Verbindungen durch Bildung von Heteropolyblau sind bekannt. In "Spot Tests in Organic Analysis" von F. Feigl, Elsevier Publishing Company, 5. Auflage, 1956, Seite 128 und 129 wird beschrieben, daß 12-Molybdophosphorsäure durch viele reduzierende Substanzen zu Molybdänblau reduziert wird. Da diese Nachweisreaktion unspezifisch ist, wird zur Erzielung einer gewissen Selektivität empfohlen, die zu untersuchende Probe alkalisch zu machen und anschließend mit Ether zu extrahieren. Der Etherextrakt enthält dann vor allem aromatische Stickstoffbasen und etherlösliche neutrale Substanzen. Saure Substanzen bleiben in der wässrigen Phase gelöst.

Aus P. B. Issopoulos, Pharm. Acta Helv. 64, 82 (1989) ist bekannt, daß bestimmte Arzneimittel, die eine o-Hydrochinonstruktur enthalten, wie z. B. Methyldopa in schwefelsaurer Lösung reduzierend auf Molybdophosphorsäure einwirken und zur Bildung von Molybdänblau führen.

In M. L. Matheke et al., Clin. Chem. 33, 2109 - 2110 (1987) wird der Einsatz von Phosphorwolframsäure zum Nachweis von Harnsäure beschrieben. Die reduktive Bildung von Wolframblau dient als Indikator für die Anwesenheit von Harnsäure. Die Nachweisreaktion wird durch die Anwesenheit reduzierend wirkender Arzneimittel gestört.

Aus B. Klein et al., Clin. Chem. 12, 816 - 823 (1966) ist eine Bestimmung von Glucose in Serum oder Plasma bekannt, die auf folgender Reaktionssequenz beruht:
Glucose wird durch Kaliumhexacyanoferrat (III) oxidiert, wobei das gebildete Kaliumhexacyanoferrat (II) reduzierend auf Phosphormolybdänsäure wirkt und zu Molybdänblau-Bildung führt. Da Serum und Plasma unterschiedliche Mengen an Harnsäure, Arzneimitteln oder sonstigen reduzierend wirkenden Substanzen, wie beispielsweise Bilirubin und Glutathion enthalten können, ist deren störender Einfluß bei diesem verfahren vorprogrammiert. In Chemical Abstracts, 100, Seite 208, 26068 m wird der Nachweis von SnCl₂ durch ein Prenylamoniumsalz von Molybdophosphaten oder Molybdosilikaten durch Reduktion in DMF zu Heteropolyblau beschrieben.

Der Nachteil aller bisher bekannten analytischen Methoden, die die reduktive Bildung von Heteropolyblau aus Heteropolysäuren nutzen, liegt vor allem in der Unspezifität der jeweiligen Nachweisreaktion. Sehr viele reduzierende Substanzen können störend wirken, da sie ebenfalls zur Heteropolyblau-Bildung führen. Zusätzlich sind Heteropolysäuren nur unter sauren Bedingungen stabil. Dies schränkt den Anwendungsbereich sehr stark ein. Insbesondere die Kopplung der Heteropolyblau-Bildung mit enzymatischen Reaktionsschritten zum spezifischen Nachweis und zur spezifischen Bestimmung von Substanzen ist bisher nicht bekannt. Gerade für den Nachweis von Bestandteilen von Körperflüssigkeiten wie Blut, Serum, Plasma, Harn etc. sind enzymatische Bestimmungsverfahren aber häufig notwendig.

Aufgabe der vorliegenden Erfindung war es deshalb, die Heteropolyblau-Bildung als Nachweis- und Bestimmungsreaktion von Substanzen, insbesondere von Substanzen in Körperflüssigkeiten und insbesondere in Kombination mit enzymatischen Reaktionsschritten zu nutzen. Dabei sollten reduzierend wirkende Begleitsubstanzen nicht störend wirken und die Nachweis- und Bestimmungsreaktion sollte bei für enzymatische Reaktionen erforderlichem pH-Wert ablaufen. Außerdem sollte die Nachweis- und Bestimmungsreaktion schnell durchgeführt werden können.

Diese Aufgabe wird durch Maßnahmen gelöst, wie sie in den Ansprüchen charakterisiert sind.

Es wurde gefunden, daß ein schwer lösliches Salz einer Heteropolysäure vorteilhaft zur Bestimmung eines Analyts verwendet werden kann, gekennzeichnet dadurch, daß der Analyt ein elektronenreiches aromatisches Amin ist oder er zusammen mit einer weiteren Substanz zu einem solchen führt.

Das erfindungsgemäße Verfahren zur Bestimmung eines Analyts mittels Heteropolyblau-Bildung ist dadurch gekennzeichnet, daß der Analyt mit einer Substanz umgesetzt wird, die zur Bildung eines elektronenreichen aromatischen Amins führt, das mit einem schwer löslichen Salz einer Heteropolysäure kontaktiert wird. Selbstverständlich kann das erfindungsgemäße Verfahren auch zur Bestimmung eines elektronenreichen aromatischen Amins selbst herangezogen werden, indem eine Lösung dieses Amins mit einem schwer löslichen Salz einer Heteropolysäure kontaktiert wird.

Die der Erfindung zugrundeliegende Aufgabe wird außerdem gelöst durch die Verwendung eines schwer löslichen Salzes einer Heteropolysäure zur Herstellung eines Mittels zur Bestimmung eines elektronenreichen, aromatischen Amins durch Heteropolyblau-Bildung.

Erfindungsgemäß wird ein Mittel zur Bestimmung eines Analyts durch Heteropolyblau-Bildung bereitgestellt, das dadurch gekennzeichnet ist, daß es eine Substanz enthält, die mit dem Analyt zu einem elektronenreichen aromatischen Amin führt und außerdem ein schwer lösliches Salz einer Heteropolysäure oder die ein solches Salz bei Kontakt ergebenden Substanzen. Soll das erfindungsgemäße Mittel direkt zur Bestimmung eines elektronenreichen aromatischen Amins dienen, genügt es, wenn es ein schwer lösliches Salz einer Heteropolysäure oder die ein solches Salz bei Kontakt ergebenden Substanzen in flüssigem Medium oder in trägergebundener Form enthält.

Gemäß der vorliegenden Erfindung kann ein schwer lösliches Salz einer Heteropolysäure zur Bestimmung eines elektronenreichen, aromatischen Amins verwendet werden. Als schwer lösliches Salz einer Heteropolysäure im Sinne der vorliegenden Erfindung wird vor allem ein solches Salz einer Heteropolysäure verstanden, das in Wasser oder wässrigen Medien, wie Puffer oder Körperflüssigkeiten, wie beispielsweise Blut, Plasma, Serum, Harn oder Speichel gar nicht oder nur sehr wenig löslich ist und dies auch unter den Test- und Farbbildungsbedingungen bleibt. Insbesondere ist das Salz einer Heteropolysäure
so schlecht löslich, daß die in einer flüssigen Probe maximal lösbare Menge allein nicht zur Bestimmung des darin enthaltenen Analyts ausreichen würde.

Überraschenderweise hat es sich gezeigt, daß ein solches schwer lösliches Salz einer Heteropolysäure nicht nur im sauren, sondern auch im neutralen und basischen pH-Bereich, insbesondere bis etwa pH 10 stabil ist. Es kann somit in pH-Bereichen eingesetzt werden, wo die meisten Enzyme aktiv sind und damit zur Bildung von Heteropolyblau in Kombination mit enzymatischen Reaktionen herangezogen werden. Vor allem im ungelösten Zustand ist ein erfindungsgemäßes schwer lösliches Salz einer Heteropolysäure auch bei erhöhter Temperatur stabil. Trotz der Schwerlöslichkeit hat es sich erwiesen, daß ein Analyt mit einem erfindungsgemäßen Salz einer Heteropolysäure sehr schnell, d. h. innerhalb weniger Sekunden bis einiger weniger Minuten durch Heteropolyblaubildung nachgewiesen und bestimmt werden kann. Hierbei ist eine selektive Bestimmung ohne Störung durch andere an sich reduzierend wirkende Substanzen möglich. Dies erlaubt eine empfindliche Meßmethode, die insbesondere auch wegen des breiten Absorptionsmaximums des gebildeten Heteropolyblaus, das von etwa 550 bis über 1100 nm hinaus reicht, keine besonderen Anforderungen an Meßgeräte stellt und auch visuell zu verfolgen ist.

Erfindungsgemäß einsetzbare Salze von Heteropolysäuren sind Salze von Heteropolysäuren des Molybdäns, des Molybdäns und Wolframs, des Vanadiums und Molybdäns oder des Vanadiums und Molybdäns und Wolframs mit Phosphor, Arsen, Silicium oder Germanium als Heteroatomen. Besonders bevorzugt sind Heteropolysäuren des Molybdäns mit Phosphor oder Arsen. Phosphor ist als Nichtmetallatom ganz besonders bevorzugt. Als Metallatom ist Molybdän ganz besonders bevorzugt. Hervorragend geeignet sind schwer lösliche Salze von 12-Molybdophosphorsäure, 18-Molybdodiphosphorsäure, 12-Molyboarsensäure, 18-Molybdodiarsensäure, 11-Molybdo-1-vanadophosphorsäure, 10-Molybdo-2-vanadophosphorsäure und 9-Molybdo-3-vanadophosphorsäure, wobei 18-Molybdodiphosphorsäure aufgrund seiner hohen Farbausbeute bei Reduktion zu Molybdänblau besonders bevorzugt ist.

Schwer lösliche Salze von Heteropolysäuren sind solche, deren Kationen größer sind als das Ammoniumion, NH₄⁺. Bevorzugte Kationen können dargestellt werden durch die allgemeine Formel I

R¹R²R³R⁴X⁺ (I)

in der
- R¹, R², R³, R⁴: gleich oder verschieden sind und einen Alkyl-, Aryl- oder Aralkylrest bedeuten, oder
Wasserstoff, wenn nicht alle Reste gleich sind oder
wobei zwei Reste zusammen einen Alkylenrest bilden, und
- X: ein Phosphor- oder Stickstoffatom bedeutet.

In Alkyl- und Aralkylresten bedeutet Alkyl einen 1-22 Kohlenstoffatome, vorzugsweise 1-6 Kohlenstoffatome enthaltenden, geradkettigen oder verzweigten Rest. Aryl in Aryl- oder Aralkylresten bedeutet einen aromatischen Rest aus 6 bis 10 Kohlenstoffatomen. Besonders bevorzugt sind Phenyl oder Naphthyl.

Als Aralkylrest besonders bevorzugt ist die Benzylgruppe.

Ein Alkylenrest ist eine gesättigte oder ungesättigte Kohlenwasserstoffkette aus 4-6, bevorzugt 4 oder 5 Kohlenstoffatomen, die mit beiden Enden an X gebunden ist. Kationen der allgemeinen Formel I können zwei solcher Alkylenreste enthalten. Vorzugsweise wird jedoch lediglich ein Alkylenrest vorhanden sein.

In der allgemeinen Formel I bedeutet X vorzugsweise ein Stickstoffatom. Ebenso können bevorzugte Kationen in schwer löslichen Salzen von Heteropolysäuren auch solche aus der Gruppe der ein quaternäres N-Atom enthaltenden Stickstoff-Heteroaromaten sein. Beispielhaft seien genannt Pyridin oder Chinolin, die an ihrem Stickstoffatom einen Alkyl-, Aryl- oder Aralkylrest tragen, wobei für die Definition dieser Reste die zuvor für die Reste R¹, R², R³, R⁴ in der allgemeinen Formel I gegebene ebenfalls zutrifft.

Ein erfindungsgemäßes schwerlösliches Salz einer Heteropolysäure kann beispielsweise durch Umsetzung einer Heteropolysäure mit einer entsprechenden basischen Substanz erhalten werden. In der Regel wird hierzu mindestens eine Substanz als Lösung, vorzugsweise als wässrige Lösung eingesetzt werden. Es können aber auch beide Salzkomponenten in fester Form einer Flüssigkeit, insbesondere einer wässrigen Flüssigkeit zugegeben werden oder umgekehrt.

Als Analyt wird eine zu bestimmende Substanz bezeichnet. Besonders geeignet hat sich die Erfindung für Substanzen erwiesen, die in einer Flüssigkeit, insbesondere wässriger Flüssigkeit, gelöst vorliegen. Besonders vorteilhaft kann ein schwer lösliches Salz einer Heteropolysäure zur Bestimmung von Substanzen in Körperflüssigkeiten, wie beispielsweise Blut, Plasma, Serum, Harn oder Speichel verwendet werden. Als mögliche Analyten in diesem Sinn sind beispielsweise Glucose, Cholesterin, Lactat, NADH oder Ethanol zu nennen. Grundsätzlich können erfindungsgemäß alle diejenigen Verbindungen bestimmt werden, die zusammen mit einer oder mehreren anderen Verbindungen zu solchen Substanzen umgesetzt werden können oder die selbst solche Substanzen sind, die bezüglich ihres Redoxpotentials und bezüglich ihrer Kinetik in der Lage sind, ein schwer lösliches Salz einer Heteropolysäure innerhalb weniger Sekunden bis einiger Minuten, vorzugsweise in weniger als drei Minuten zu Heteropolyblau zu reduzieren. Überraschenderweise hat es sich gezeigt, daß elektronenreiche aromatische Amine dies vermögen. Vor allem ist es überraschend, daß hierbei eine selektive Bestimmung ohne Störung durch andere an sich reduzierend wirkende Verbindungen möglich ist.

Unter einem elektronenreichen aromatischen Amin wird eine Verbindung verstanden, die elektronenreicher als Anilin ist und damit ein stärkeres Reduktionsmittel als Anilin darstellt. Elektronenreiche aromatische Amine besitzen ein Redoxpotential von weniger als 0,6 V, bevorzugt kleiner als 0,45 V, gegen eine Normalwasserstoffelektrode. Die Größe des Redoxpotentials allein ist jedoch nicht entscheidend. Wichtig ist außerdem, daß die entsprechende Substanz schnell, d. h. innerhalb von weniger als etwa drei Minuten in der Lage ist, ein schwerlösliches Salz einer Heteropolysäure zu Heteropolyblau zu reduzieren. In Frage kommen beispiels-weise alle Anilinderivate, die einen oder mehrere +I oder/und +M-Substituenten, wie Hydroxy-, Alkyl-, Alkoxy-, Aryloxy-, Alkylthio-, Arylthio-, Amino-, Monoalkylamino- und Dialkylaminoreste tragen.

Alkyl-, Alkoxy-, Alkylthio-, Monoalkylamino- und Dialkylaminoreste sind Reste, in denen Alkyl einen Kohlenwasserstoffrest mit 1-6 Kohlenstoffatomen darstellt, der seinerseits durch eine Hydroxygruppe, eine gegebenenfalls ein- oder mehrfach durch Alkyl mit 1-6 Kohlenstoffatomen substituierte Aminogruppe, PO₃H₂, SO₃H oder CO₂H substituiert sein kann. Die Säurereste PO₃H₂, SO₃H und CO₂H können als solche oder in Salzform als Ammonium-, Alkali- oder Erdalkalisalze vorliegen.

Aryloxy- und Arylthioreste sind Reste mit 6-10 Kohlenstoffatomen, wobei Phenoxy- und Phenylthioreste besonders bevorzugt sind.

Unter Anilinderivaten werden auch Verbindungen verstanden, die eine unsubstituierte oder durch Alkyl ein- oder mehrfach substituierte Aminogruppe an einem aromatischen Ringsystem tragen, das mit einem oder mehreren aromatischen oder/und alicyclischen Ringen anelliert ist. Als aromatische Ringe kommen hierbei sowohl kohlenstoffaromatische Systeme als auch Heteroaromaten in Frage. Beispiele sind anellierte Benzol- oder Naphthalinringe oder ein anellierter Pyridinring.

Unter alicyclischen Ringen werden gesättigte oder ungesättigte Cycloaliphaten mit 5-7 Kohlenstoffatomen, vorzugsweise 5 oder 6 Kohlenstoffatomen verstanden.

Mögliche Alkylsubstituenten der Aminogruppe können Kohlenwasserstoffreste mit 1 - 6 Kohlenstoffatomen sein, die ihrerseits durch eine Hydroxygruppe, eine gegebenenfalls ein- oder mehrfach durch Alkyl mit 1 - 6 Kohlenstoffatomen substituierte Aminogruppe, PO₃H₂, SO₃H und CO₂H substituiert sein können. Die Säurereste PO₃H₂, SO₃H und CO₂H können als solche oder in Salzform als Ammonium-, Alkali- oder Erdalkalisalze vorliegen.

Besonders geeignet als elektronenreiche aromatische Amine sind Verbindungen der allgemeinen Formel II 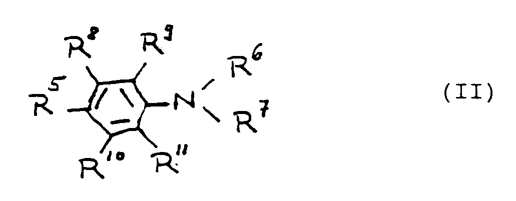
in der
- R⁵: Hydroxy oder Amino bedeutet, wobei die Aminogruppe gegebenenfalls ein- oder zweifach durch einen Alkylrest substituiert ist und der Alkylrest seinerseits gegebenenfalls durch eine Hydroxygruppe, eine gegebenenfalls ein- oder mehrfach durch Alkyl substituierte Aminogruppe, PO₃H₂, SO₃H oder CO₂H substituiert ist,
- R⁶ und R⁷: die beide gleich oder verschieden sind, Wasserstoff oder Alkyl darstellen, wobei der Alkylrest gegebenenfalls durch eine Hydroxygruppe, eine gegebenenfalls ein- oder mehrfach durch Alkyl substituierte Aminogruppe, PO₃H₂, SO₃H oder CO₂H substituiert ist und
- R⁸, R⁹, R¹⁰ und R¹: ¹ gleich oder verschieden sind und Wasserstoff,
Alkyl, Alkoxy, Alkylthio, Aryloxy, Arylthio, Halogen, Carboxy, Carboxyalkyl oder Alkoxycarbonyl bedeutet.

Alkylreste und "Alkyl" in Alkylthio, Carboxyalkyl- und Alkoxycarbonylresten sind Kohlenwasserstoffreste mit 1-6 Kohlenstoffatomen. Besonders bevorzugt sind Reste mit 1-3 Kohlenstoffatomen.

Alkoxyreste sind ebenfalls Kohlenwasserstoffreste mit 1 - 6 Kohlenstoffatomen. Besonders bevorzugt sind Reste mit 1 - 3 Kohlenstoffatomen. Aryloxy- und Arylthioreste sind Reste mit 6 - 10 Kohlenstoffatomen, wobei Phenoxy- und Phenylthioreste besonders bevorzugt sind. Halogen bedeutet Fluor, Chlor, Brom oder Jod. Chlor und Brom sind bevorzugte Halogensubstituenten.

Säurereste PO₃H₂, SO₃H oder CO₂H können als solche oder in Salzform als Ammonium-, Alkali- oder Erdalkalisalz vorliegen.

Ammoniumsalze sind solche, die das Ammoniumion, NH₄⁺ enthalten oder solche, die ein- oder mehrfach durch Alkyl-, Aryl- oder Aralkylreste substituierte Ammoniumkationen enthalten. Alkyl in Alkyl- und Aralkylresten bedeutet einen Kohlenwasserstoffrest mit 1-6 Kohlenstoffatomen. Aryl in Aryl- und Aralkylresten ist ein 6-10 Kohlenstoffatome zählendes aromatisches Ringsystem, wobei Phenyl bevorzugt ist. Ein bevorzugter Aralkylrest ist Benzyl.

Alkalisalze sind vorzugsweise solche des Lithiums, Natriums oder Kaliums. Erdalkalisalze sind vorzugsweise solche des Magnesiums oder Calciums.

Elektronenreiche aromatische Amine können unmittelbar mit einem schwerlöslichen Salz einer Heteropolysäure durch Bildung von Heteropolyblau bestimmt werden. Es können aber auch Substanzen bestimmt werden, die durch chemische oder enzymatische Reaktion mit einer weiteren Substanz diese in stöchiometrischer Weise zu einem elektronenreichen aromatischen Amin umsetzen. Beispielsweise können als Reaktionspartner solche Substanzen verwendet werden, die das Grundgerüst eines elektronenreichen aromatischen Amins bereits in sich bergen und bei denen es durch eine chemische oder enzymatische Reaktion mit dem zu bestimmenden Analyt freigesetzt werden kann. Vorzugsweise werden hierunter solche Verbindungen verstanden, die durch Hydrolyse zu einem elektronenreichen aromatischen Amin führen. Als Beispiele seien Amide, Ester oder Glycoside von elektronenreichen aromatischen Aminen genannt. Analyte, die so bestimmt werden können, sind vorzugsweise Enzyme, die die Umsetzung entsprechender Substrate in elektronenreiche aromatische Amine katalysieren, insbesondere Hydrolasen wie Amidbindungen und/oder Peptidbindungen spaltende Enzyme, Esterbindungen - seien es Carbonsäure-, Phosphorsäure- oder Schwefelsäurebindungen - spaltende Enzyme und glykosidische Bindungen spaltende Enzyme, außerdem auch Transferasen, die die Übertragung von Gruppen katalysieren, wie beispielsweise γ-Glutamyltransferase.

Weiter können beispielsweise auch Substanzen bestimmt werden, die enzymatisch oxidiert werden können und wobei als Elektronenakzeptoren solche Verbindungen eingesetzt werden, die durch Reduktion elektronenreiche aromatische Amine ergeben. Vor allem für Substanzen in Körperflüssigkeiten, wie Blut, Plasma, Serum, Harn oder Speichel sind eine Vielzahl von Oxidoreductasen bekannt, die jeweils spezifisch bestimmte Substanzen erkennen und in Anwesenheit eines funktionierenden Elektronenacceptors diese Substanzen oxidieren.

Als Beispiele seien flavinabhängige Oxidasen wie L- und D-Aminosäure-Oxidase, Cholesterin-Oxidase, Glucose-Oxidase, Glycerin-3-phosphat-Oxidase, Lactat-Oxidase oder Pyruvat-Oxidase und nicht-NAD(P)-abhängige Dehydrogenasen wie die Pyrrolochinolin-chinonabhängige Glucose-Dehydrogenase genannt oder auch Diaphorase (NADH: dye-Oxidoreductase).

Im Falle von Oxidoreduktasen, insbesondere bei Oxidasen und nicht-NAD(P)-abhängigen Dehydrogenasen sind Elektronenakzeptoren für enzymatische Oxidationen einsetzbar, die durch die enzymatische Oxidation des Enzymsubstrats zu einem elektronenreichen aromatischen Amin reduziert werden. So können dann solche Substanzen, die enzymatisch oxidiert werden, durch Kontaktierung des entstehenden elektronenreichen aromatischen Amins mit einem schwer löslichen Salz einer Heteropolysäure durch Heteropolyblau-Bildung nachgewiesen und bestimmt werden.

Als vorteilhafte Elektronenakzeptoren im erfindungsgemäßen Sinn sind in diesem Zusammenhang insbesondere aromatische Nitrosoverbindungen, Oxime und Hydroxylamine zu nennen. Besonders bevorzugt sind aromatische Nitrosoverbindungen und Oxime. Ganz besonders bevorzugt sind insbesondere solche Nitrosoverbindungen und Oxime wie sie in der Europäischen Patentanmeldung Nr. EP-A-0354 441 beschrieben sind.

Zur Durchführung der erfindungsgemäßen Bestimmung eines Analyts durch Heteropolyblau-Bildung genügt es, den Analyt wie vorstehend beschrieben mit einer Substanz zur Reaktion zu bringen, zu einem elektronenreichen aromatischen Amin umzusetzen und mit einem schwer löslichen Salz einer Heteropolysäure zu kontaktieren. Wenn das elektronenreiche aromatische Amin selbst der unmittelbar zu bestimmende Analyt ist, wird dieses ohne vorhergehende chemische oder enzymatische Reaktionen mit einem schwer löslichen Salz einer Heteropolysäure kontaktiert.

In der Regel wird zumindest das schließlich auf das Salz der Heteropolysäure einwirkende elektronenreiche aromatische Amin in gelöster Form vorzugsweise in wässriger Lösung, beispielsweise Wasser, Puffer oder Körperflüssigkeit, vorliegen. Vorteilhafterweise wird dann, wenn der zu bestimmende Analyt nicht selbst ein elektronenreiches aromatisches Amin ist, die zusammen mit dem Analyt zur Erzeugung eines elektronenreichen aromatischen Amins notwendige Verbindung ebenfalls in wässriger Flüssigkeit löslich sein. Sie kann der Probe vor der Kontaktierung mit einem schwer löslichen Salz einer Heteropolysäure zugegeben werden. Sie kann aber auch erst zusammen mit dem schwer löslichen Salz einer Heteropolysäure oder sogar erst zuletzt mit der zu untersuchenden Probe in Kontakt gebracht werden. Welche Reihenfolge gewählt wird, ist vom Einzelfall abhängig und kann von dem Fachmann für die jeweils durchzuführende Analytbestimmung aufgrund seines allgemeinen Fachwissens oder durch wenige optimierende Versuche entschieden werden. Die zur Erzeugung eines elektronenreichen aromatischen Amins notwendige Substanz kann einer wässrigen Probe fest oder in gelöster Form zugegeben werden.

Die zusammen mit dem zu bestimmenden Analyt ein elektronenreiches aromatisches Amin ergebende Substanz muß in mindestens so großer Menge mit der Probe kontaktiert werden, daß der gesamte Analyt zur Reaktion gebracht werden kann. Vorzugsweise wird ein 2 - 10facher Überschuß eingesetzt.

Das schwer lösliche Salz einer Heteropolysäure kann als feste Substanz mit der zu untersuchenden Probe in Kontakt gebracht werden. Es kann aber auch als Suspension in einer Flüssigkeit, vorzugsweise wässriger Flüssigkeit, vorliegen, wobei dann zur Durchführung der Bestimmung die Suspension mit der zu untersuchenden Probe gemischt wird. Bei Anwesenheit des zu bestimmenden Analyts bildet sich in Wasser schwer lösliches Heteropolyblau, das aufgrund seiner intensiven Farbe erkennbar ist. Zur quantitativen Bestimmung ist es möglich, das in Wasser schwer lösliche Heteropolyblau sowie unumgesetztes Heteropolysäuresalz von der Flüssigkeit abzutrennen, beispielsweise durch Zentrifugation, anschließend in einem geeigneten Lösungsmittel, wie beispielsweise Dimethylsulfoxid aufzulösen und photometrisch gegebenenfalls unter Heranziehung von Standardlösungen oder Eichkurven die Konzentration des Heteropolyblau-Farbstoffs zu messen und damit letztendlich die Konzentration des nachzuweisenden Analyts zu bestimmen.

Das schwer lösliche Salz einer Heteropolysäure muß in so großer Menge anwesend sein, daß das in der Probe vorliegende oder gebildete elektronenreiche aromatische Amin zu einer Heteropolyblau-Bildung führt, die in einen quantitativen Zusammenhang mit der Menge des zu bestimmenden Analyt bzw. des elektronenreichen aromatischen Amins gesetzt werden kann. Grundsätzlich muß deshalb soviel schwer lösliches Salz einer Heteropolysäure eingesetzt werden, daß bis zur höchsten relevanten Konzentration des elektronenreichen aromatischen Amins eine Erhöhung der Aminmenge zu einer Zunahme der Heteropolyblaumenge führt.

Ein erfindungsgemäßes Mittel zur Bestimmung eines Analyts durch Heteropolyblau-Bildung enthält eine Substanz, die mit dem Analyt zu einem elektronenreichen aromatischen Amin führt und ein schwer lösliches Salz einer Heteropolysäure. Ein solches Mittel kann beispielsweise als Suspension oder als Lyophilisat, Pulvermischung oder als Tablette verpreßt zum Einsatz gebracht werden. Die Bestandteile können nebeneinander oder getrennt vorliegen, wobei jeder der Bestandteile in seiner zweckmäßigsten Form verarbeitet sein kann. Es ist durchaus auch möglich, daß ein erfindungsgemäßes Mittel nicht ein einsatzbereites schwer lösliches Salz einer Heteropolysäure, sondern lediglich die zur Bereitung eines solchen Salzes notwendigen Komponenten, nämlich beispielsweise eine Heteropolysäure und eine kationische oder basische Verbindung, die beide erst unmittelbar vor der erfindungsgemäßen Bestimmungsreaktion kontaktiert werden und dann erst das entsprechende Salz ergeben, getrennt enthält. Besonders vorteilhaft ist es, wenn das erfindungsgemäße Mittel ein schwer lösliches Salz einer Heteropolysäure in feinverteilter Form, so daß es eine große reaktive Oberfläche aufweist, beinhaltet. Gegebenenfalls kann das erfindungsgemäße Mittel auch noch weitere Reagenzien, wie Puffer und Hilfsstoffe, wie beispielsweise Netzmittel, Stabilisatoren, Gerüstbildner usw. enthalten. Ist der zu bestimmende Analyt selbst ein elektronenreiches aromatisches Amin, benötigt man für das erfindungsgemäße Mittel selbstverständlich keine Substanz, die erst mit dem Analyt zu einem elektronenreichen aromatischen Amin führt.

Ganz besonders vorteilhaft gestaltet sich die Durchführung der erfindungsgemäßen Bestimmung eines Analyts mit einem sogenannten Trockentest. Vorrichtungen, die zur Durchführung eines Trockentestes geeignet sind, sind dem Fachmann beispielsweise aus EP-A-0 016 387, DE-A-32 47 608, EP-A-0 262 445 oder EP-A-0 256 806 bekannt. Sie können als Testträger bezeichnet werden. Hierbei liegen die zur Durchführung eines Tests erforderlichen Reagenzien in trockener, d. h. nicht in einer Flüssigkeit gelösten, Form beispielsweise in oder auf saugfähigem Material, wie beispielsweise Papier, einem offenen Film gemäß EP-A-0 016 387, einem Glasfaservlies oder einer porösen Kunststoffmembran, um nur einige mögliche Materialien zu nennen, oder in oder auf einem quellfähigen Material, wie z. B. einem entsprechenden Kunststoffilm, Gelatine oder Cellulose, vor.

Bei Aufgabe der Probenflüssigkeit auf den Testträger oder Eintauchen des Testträgers in die Probenflüssigkeit bildet sich in dem Testträger ein flüssiges Milieu aus, innerhalb dessen die Nachweisreaktion abläuft. Die durch die Reaktion verursachte Farbbildung kann visuell oder photometrisch, z. B. reflexionsphoto-metrisch ausgewertet werden.

Zur Herstellung des erfindungsgemäßen Mittels in trägergebundener Form wird ein geeignetes Trägermaterial, wie Filterpapier, Cellulose oder Kunstfaservlies mit Lösungen oder/und Suspensionen der erforderlichen, zur Herstellung von Testträgern verwendeten Reagenzien in leicht flüchtigen Lösungsmitteln, wie z. B. Wasser, Methanol, Ethanol oder Aceton imprägniert. Dies kann in einem Imprägnierschritt erfolgen. Oft ist es zweckmäßig, die Imprägnierung in mehreren Schritten durchzuführen, wobei Lösungen oder/und Suspensionen eingesetzt werden, die jeweils einen Teil der Bestandteile des fertigen Mittels enthalten. So kann beispielsweise in einem ersten Schritt aus einer Suspension das schwer lösliche Salz einer Heteropolysäure und in einem zweiten Schritt aus einer Lösung die Substanz, die mit dem zu bestimmenden Analyt zu einem elektronenreichen, aromatischen Amin führt sowie gegebenenfalls Puffer und andere Zusatzstoffe enthält, auf ein Trägermaterial aufgebracht werden. Das so behandelte Trägermaterial kann als solches verwendet werden oder in an sich bekannter Weise an Griffe oder auf steife Kunststoffolien geklebt werden, um besser handhabbar zu sein.

Statt einer mehrmaligen Imprägnierung des selben Trägermaterials können die Reagenzien des erfindungsgemäßen Mittels auch auf verschiedene Trägermaterialien verteilt werden, die bei Durchführung der Analytbestimmung miteinander in einen Flüssigkeitsaustausch ermöglichenden Kontakt gebracht werden.

Zur Herstellung von trägergebundenen Testmitteln können anstelle einer Imprägnierung auch aus Filmbildnern, das heißt aus Polymeren oder aus Polymerdispersionen, Lösungen hergestellt werden, die so viskos sind, daß sich aus ihnen nach den bekannten Herstellungsverfahren, wie Rakeln, Rollcoating etc. Filme herstellen lassen. In diese Lösungen werden die Reagenzien und gegebenenfalls Puffersubstanzen und Hilfsstoffe eingearbeitet. Die Beschichtungsmassen werden auf Trägerfolien aufgebracht, getrocknet und die fertigen Filme, beispielsweise zu Teststreifen verarbeitet.

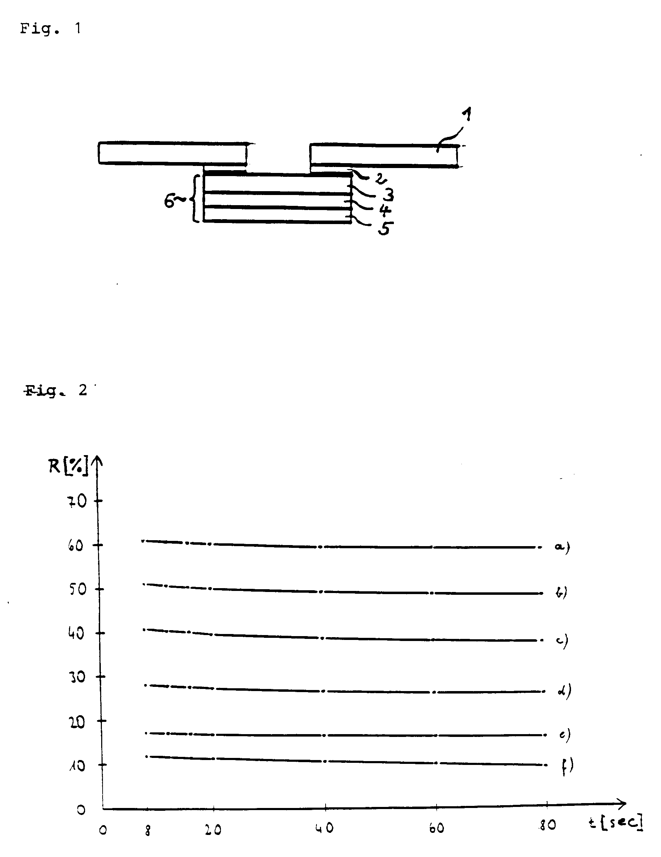

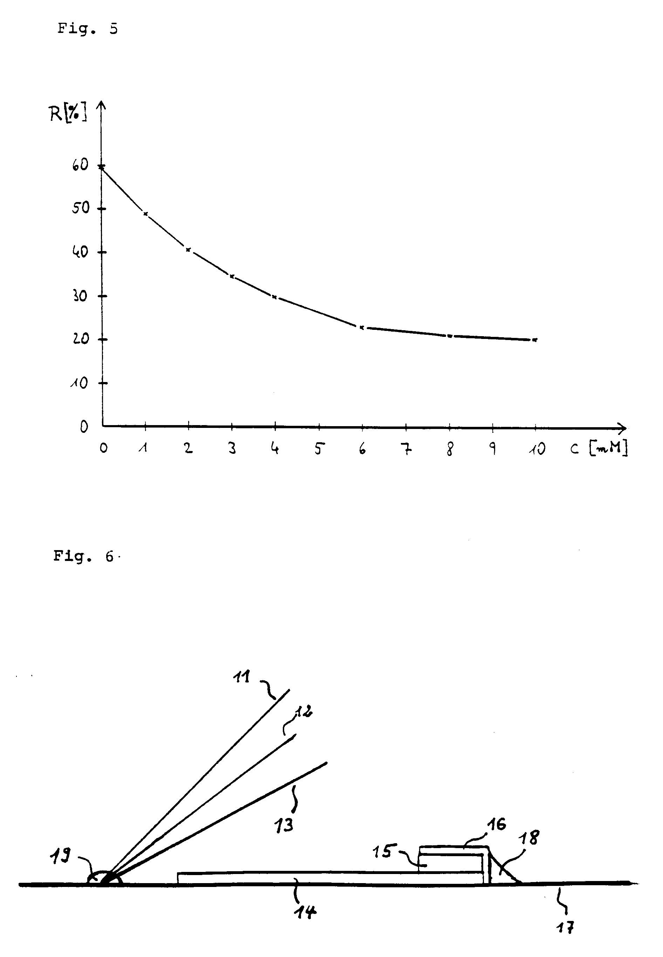

Beispiele für bevorzugte Testträger sind in Fig. 1 und 6 dargestellt. Die übrigen Abbildungen 2 - 5 und 7 zeigen Schaubilder, die Abhängigkeiten der Reflexion in Prozent von Zeit, Wellenlänge oder Analytkonzentration angeben, die mit Testträgern gemäß Fig. 1 bzw. Fig. 6 erhalten wurden.

Im einzelnen zeigen:
- Fig. 1:: einen Querschnitt durch einen bevorzugten Testträger
- Fig. 2:: ein Diagramm, das die Abhängigkeit der Reflexion in Prozent von der Zeit in Sekunden nach Probenaufgabe auf einen Testträger gemäß Fig. 1 für Proben a) - f) mit jeweils unterschiedlichen Glucosekonzentrationen zeigt.
- Fig. 3:: ein Diagramm, das die Abhängigkeit der Reflexion in Prozent von der Meßwellenlänge in Nanometern für Proben a) - f) mit unterschiedlichen Glucosekonzentrationen bei Verwendung eines Testträgers gemäß Fig. 1 zeigt.
- Fig. 4:: ein Diagramm, das die Abhängigkeit der Reflexion in Prozent von der Glucosekonzentration bei Messung mit einem Testträger gemäß Fig. 1 widergibt.
- Fig. 5:: ein Diagramm, das die Abhängigkeit der Reflexion in Prozent von der NADH-Konzentration bei Messung mit einem Testträger gemäß Fig. 1 zeigt.
- Fig. 6:: einen Querschnitt durch einen weiteren bevorzugten Testträger.
- Fig. 7:: ein Diagramm, das die Abhängigkeit der Reflexion in Prozent von der Ethanolkonzentration bei Messung mit einem Testträger gemäß Fig. 6 widergibt.

Die nachfolgenden Beispiele zeigen einige der möglichen Verfahrensvarianten zur Bestimmung eines Analyts durch Heteropolyblau-Bildung. Sie sollen jedoch keine Beschränkung der Erfindung auf diese Ausführungsformen darstellen. Die Figuren sind in den Beispielen näher erläutert.

### Beispiel 1

### Nachweis von Glucose mit 18-Molybdodiphosphat

Es wird ein Testträger gemäß Figur 1 hergestellt. Er besteht aus einer 350 »m dicken Polyesterfolie (Melinex, ICI, Frankfurt, Bundesrepublik Deutschland) als Trägerfolie (1) mit den Maßen 2 x 3 cm. Im Zentrum der Folie befindet sich ein Loch mit 6 mm
Durchmesser. Mittels Transferkleber (2) ist der Reagenzträger (6) bestehend aus einer 200 »m dicken transparenten Polycarbonat-Folie (5) (Pokalon, Lonza, Rheinfelden, Bundesrepublik Deutschland), einer ersten Reagenzschicht (4) und einer zweiten Reagenzschicht (3) so über dem Loch in der Trägerfolie befestigt, daß eine durch dieses Loch aufgegebene Probenflüssigkeit zuerst mit der zweiten Reagenzschicht in Kontakt kommt. Der Reagenzträger hat die Maße 2 x 1 cm.

Die Herstellung des Reagenzträgers (6) erfolgt folgendermaßen:
Erste Reagenzschicht:
113 g bidestilliertes Wasser,
36,3 g 2 Gew.-% Xanthan (Keltrol F, Kelco, Oklahoma, USA) in 0,2 M Citrat-Puffer, pH 7,0
69 g Propiofan 70 D (BASF, Ludwigshafen, Bundesrepublik Deutschland)
15 ml 15 Gew.-% Natrium-nonylsulfat in Wasser
6 g Polyvinylpyrrolidon (Kollidon 25, BASF, Ludwigshafen, Bundesrepublik Deutschland)
3,9 g Tetrabutylammoniumchlorid
12 g 18-Molybdodiphosphorsäure (hergestellt gemäß G. Brauer, "Handbuch der präparativen anorganischen Chemie", Enke-Verlag, Stuttgart, 1954) in 15 g Wasser
63 g Celatom MW 25 (Eagle Picher, Cincinnati, Ohio, USA)
werden zu einer homogenen Masse verrührt und in 150 »m Dicke auf die transparente Folie (5) aufgerakelt. Es wird eine Stunde bei 60 °C getrocknet.

Zweite Reagenzschicht:
20 g bidestilliertes Wasser,
16 g Titandioxid RN 56 (Kronos-Titan GmbH, Leverkusen, Bundesrepublik Deutschland)
36,3 g 2 Gew.-% Keltrol F (Kelco, Oklahoma, USA) in 0,2 M Citrat-Puffer, pH 6,0
69 g Propiofan 70 D (BASF, Ludwigshafen, Bundesrepublik Deutschland)
15 ml 15 Gew.-% Natrium-nonylsulfat in Wasser
6 g Kollidon 25 (BASF, Ludwigshafen, Bundesrepublik Deutschland)
188 g Wasser
63 g Celatom MW 25 (Eagle-Picher, Cincinnati, Ohio, USA)
400 mg N,N-Bis-(2-hydroxyäthyl)-p-nitrosoanilin x HCl in 20 g Wasser
2 g Glucoseoxidase (200 U/mg) in 12 g Wasser
werden zu einer homogenen Masse verrührt und in 400 »m Dicke auf die erste Reagenzschicht aufgerakelt. Luftblasen werden durch leichtes Anblasen entfernt und die Beschichtung wird eine Stunde bei 60 °C getrocknet.

Zum Zeitpunkt t = 0 wird Humanplasma mit a) 0 mg Glucose/dl, b) 47,5 mg Glucose/dl, c) 108,7 mg Glucose/dl, d) 200,8 mg Glucose/dl, e) 392,3 mg Glucose/dl und f) 766 mg Glucose/dl auf jeweils einen wie zuvor beschrieben hergestellten Testträger aufgegeben. Bei 950 nm wird die Remission in % gemessen. Die erste Messung erfolgt nach acht Sekunden, weitere Messungen im Vier-Sekunden-Takt. Bei Auftragung der Remission (R) in Prozent gegen die Zeit (t) in Sekunden wird ein Schaubild gemäß Figur 2 erhalten. Die Farbbildung und Remissionsabnahme ist bereits bei der ersten Messung fast vollständig. Es resultiert ein stabiler Endwert, der zu nahezu beliebiger Zeit nach dem Start gemessen werden kann.

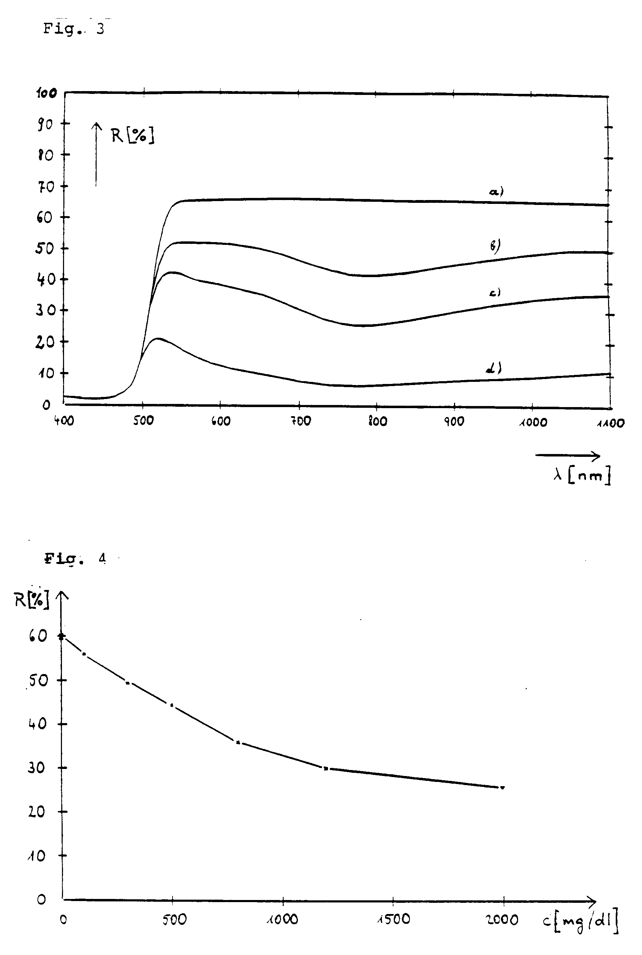

Werden mit einem Testträger gemäß Figur 1, hergestellt wie zuvor beschrieben, Proben mit einer Glucosekonzentration von a) 0 mg/dl, b) 100 mg/dl, c) 240 mg/dl und d) 800 mg/dl bei unterschiedlichen Wellenlängen vermessen und wird die Remission (R) in Prozent gegen die Wellenlänge (2) in nm in einem Schaubild aufgetragen, wird Figur 3 erhalten. Wie die dort abgebildeten Spektren verdeutlichen, können beliebige Wellenlängen zwischen 550 nm und mehr als 1100 nm zur Messung der Glucosekonzentration verwendet werden. Wegen des äußerst flachen Spektrums ist die Toleranz für Schwankungen der Meßwellenlänge sehr hoch.

### Beispiel 2

### Spezifität des Glucose-Nachweises mit 18-Molybdodiphosphat

Die in Tabelle 1 aufgeführten Störsubstanzen werden in den angegebenen Konzentrationen Humanplasma mit a) 0 mg Glucose/dl (C_{Gluc} = 0) und b) 110 mg Glucose/dl (C_{Gluc} = 110 mg/dl) zugesetzt. Wird solches Humanplasma mit einem Testträger gemäß Figur 1, wie er in Beispiel 1 hergestellt und beschrieben ist, untersucht, werden bei 660 nm die in der Tabelle 1 angegebenen Remissionswerte in Prozent (% R) gefunden.

**Tabelle 1**

| Störsubstanz | Endkonzentration | C_{Gluc} = 0 % R | C_{Gluc} = 110 mg/dl % R |
|---|---|---|---|
| keine (Vergleich | 0 | 60,3 | 43,0 |
| NaOH | 1 mM | 59,9 | 42,5 |
| Harnsäure | 10 mg/dl | 59,5 | 42,4 |
| | 20 mg/dl | 60,1 | 42,4 |
| Lactat | 100 mg/dl | 59,7 | 43,2 |
| | 300 mg/dl | 60,2 | 42,8 |
| Bilirubin | 10 mg/dl | 60,0 | 42,7 |
| | 20 mg/dl | 61,5 | 43,0 |
| Glutathion | 1 mM | 59,3 | 43,0 |
| | 5 mM | 59,4 | 43,8 |
| Methyldopa | 10 mg/dl | 59,5 | 43,1 |
| | 100 mg/dl | 59,0 | 42,1 |
| Dobesylat | 20 mg/dl | 59,4 | 41,8 |
| Gentisinsäure | 50 mg/dl | 59,6 | 43,0 |
| Aspirin | 5 mg/dl | 59,9 | 42,1 |
| | 60 mg/dl | 60,0 | 42,0 |

Unabhängig von der Glucosekonzentration in Humanplasma (0 und 110 mg/dl) werden keine signifikanten Störungen gefunden.

Mit einem Testträger gemäß Figur 1, der analog zu Beispiel 1 hergestellt wird und der mit dem dort beschriebenen Testträger bis auf den Einsatz von Tetrabutylammoniumchlorid, das für den Vergleich-Testträger weggelassen wird, identisch ist, werden sehr schlecht reproduzierbare, stark streuende Ergebnisse erhalten, weil sich die eingesetzte 18-Molybdodiphosphorsäure bei pH-Werten größer als 5,5 zersetzt. Außerdem wirken hier reduzierende Substanzen störend durch zusätzliche Farbbildung.

### Beispiel 3

### Testträger zum Nachweis von Glucose auf Basis von 12-Molybdophosphat

Wird in der Rezeptur von Beispiel 1 die 18-Molybdodiphosphorsäure ersetzt durch die gleiche Menge 12-Molybdophosphorsäure (Fluka, Buchs, Schweiz), so erhält man einen Testträger mit schwächerer Farbbildung bei Anwesenheit von Glucose, der sich besonders gut für höhere Glucosekonzentrationen eignet. Unter Verwendung von Proben unterschiedlicher aber bekannter Glucosekonzentration (C) und Messung der jeweiligen Remission (R) in Prozent bei 650 nm erhält man die Kurve gemäß Figur 4. Wird bei 950 nm gemessen, erhält man eine identische Kurve.

Ähnliche Ergebnisse werden mit 12-Molybdoarsenat (V), 18-Molybdodiarsenat (V), 11-Molybdo-1-vanadophosphat, 10-Molybdo-2-vanadophosphat und 9-Molybdo-3-vanadophosphat erhalten, die alle gemäß G. Brauer, "Handbuch der präparativen anorganischen Chemie", Enke-Verlag, Stuttgart (1954) hergestellt werden können. Der folgenden Tabelle 2 sind Name und Formel der Heteropolysäure sowie das Absorptionsmaximum des entsprechenden Heteropolyblaus zu entnehmen.

**Tabelle 2**

| Heteropolyblau aus verschiedenen Heteropolysäuren | | |
|---|---|---|
| Ausgangssubstanz | Formel | Absorptionsmax. des Heteropolyblaus |
| 12-Molybdophosphorsäure | H₃ (PMo₁₂O₄₀) x n H₂ O | 725 nm |
| Natrium-18-molybdodiphosphat | Na₆ (P₂Mo₁₈O₆₂) x 42 H₂ O | 693 nm |
| Kalium-12-molybdoarsenat (V) | K₃ (AsMo₁₂O₄₀) x n H₂ O | 840^{a} bzw. |
| | | 652 nm^{b} |
| Natrium-18-molybdodiarsenat (V) | Na₆ (As₂Mo₁₈O₆₂) x 23 H₂ O | 672 nm |
| 11-Molybdo-1-vanadophosphorsäure | H₄ (PMo₁₁VO₄₀) x n H₂ O | 665 nm |
| 10-Molybdo-2-vanadophosphorsäure | H₅ (PMo₁₀V₂O₄₀) x n H₂ O | 620 nm |
| 9-Molybdo-3-vanadophosphorsäure | H₆ (PMo₉V₃O₁₀) x n H₂ O | 780 nm |

| | | |
|---|---|---|
| a: wenig Reduktionsmittel (Glucose) | | |
| b: viel Reduktionsmittel (Glucose) | | |

### Beispiel 4

### Testträger zur Bestimmung von NAD(P)H

Wird in der Rezeptur von Beispiel 1 die Glucose-Oxidase durch die gleiche Menge Diaphorase ersetzt, so erhält man einen Testträger zum Nachweis von NAD(P)H. Aus Proben mit bekannter Konzentration an NADH und Messung der Reaktion in Prozent bei 660 nm ergibt sich die Kurve gemäß Figur 5. Diese Kurve kann als Eichkurve zur Bestimmung unbekannter NADH-Konzentrationen dienen.

Methoden zur Bildung von NAD(P)H aus NAD(P)-abhängiger Dehydrogenase, entsprechendem Substrat und NAD(P) sind bekannt. Nach entsprechender Vorreaktion kann daher der NADH-Testträger zum Nachweis von Dehydrogenase-Substraten oder zum Nachweis der Dehydrogenasen selbst genutzt werden.

### Beispiel 5

### Testträger zur Bestimmung von Ethanol mittels Alkoholdehydrogenase und Diaphorase sowie 18-Molybdodiphosphat

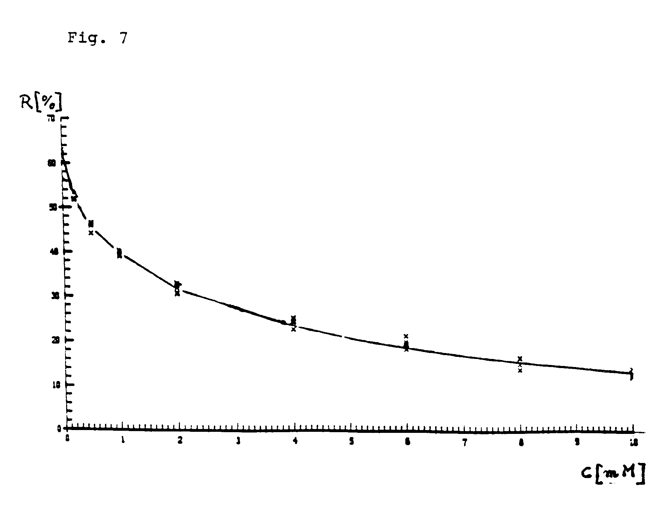

a) Herstellung des Testträgers
   Es wird ein Testträger gemäß Figur 6 hergestellt. Dieser Testträger wird so aufgebaut, daß das Indikatorsystem aus einer homogenen Suspension bestehend aus
   4 ml 0,2 M Citrat-Puffer, pH 6,0
   10 ml 10 Gew.-% Natrium-nonylsulfat in Wasser
   60 mg Tartrazin
   0,1 g N,N-bis-(2-hydroxyethyl)-p-nitrosoanilin x HCl
   6,8 ml 20 Gew.-% 18-Molybdodiphosphorsäure in Wasser
   1 g 25 Gew.-% Tetraethylammoniumchlorid in Wasser
   72 g 5 Gew.-% Kollidon 25 (BASF, Ludwigshafen, Bundesrepublik Deutschland) in 50 mM Citrat-Puffer, pH 6,0
   auf ein saugfähiges Papier (Langfaser, Schoeller, Germsbach, Bundesrepublik Deutschland) getränkt und anschließend 30 Minuten lang bei 40 °C getrocknet wird.
   Die Enzyme sind auf einem separaten Papier (Langfaser, Schoeller, Germsbach, Bundesrepublik Deutschland) untergebracht. Das Papier wird getränkt mit einer Lösung von
   50 g 0,2 M Phosphatpuffer, pH 7,0
   48 g Wasser
   2 ml 10 Gew.-% Natrium-nonylsulfat in Wasser
   6 g Diaphorase (15 U/mg Lyophilisat)
   2 g Alkoholdehydrogenase (250 U/mg Lyophilisat),
   und anschließend bei 40 °^{C} 20 Minuten lang getrocknet. Das so hergestellte Indikatorpapier (13) und Enzympapier (12) wird jeweils in 14 x 6 mm große Streifen geschnitten.
   Auf einer 350 »m dicken, 9,8 cm langen und 0,6 cm breiten Polyesterfolie (Melinex, ICI, Frankfurt, Bundesrepublik Deutschland) wird ein 16 mm langes, 6 mm breites und 0,25 mm dickes Glasfaservlies mit einem Flächengewicht von 25 g/m² als Transportvlies (14), ein 6 mm breites, 6 mm langes und 700 »m dickes Glasfaservlies mit einem Flächengewicht von 60 g/m² als Erythrozytenabtrennvlies (15) sowie ein Schutznetz (16) aus Scrynel PE280HC rot^{R} (Züricher Beuteltuchfabrik AG, Rüschlikon, Schweiz) mit den Maßen 6 x 8 mm und einer Maschenweite von 280 »m mittels eines Schmelzkleberstreifens (18) wie in Figur 6 angegeben, befestigt. Enzympapier (12) und Indikatorpapier (13) werden zusammen mit einer 200 »m dicken, 15 mm langen und 6 mm breiten transparenten Polycarbonatfolie (Pokalon, Lonza, Rheinfelden, Bundesrepublik Deutschland) (11) mittels eines Schmelzklebertropfens (19) so auf der Trägerfolie (17) befestigt, daß (11), (12) und (13) sich nicht berühren, durch Druck aber sowohl miteinander als auch in Flüssigkeitskontakt mit einer in Transportvlies (14) befindlichen Flüssigkeit gebracht werden können.
b) Bestimmung von Ethanol
   Auf den unter a) hergestellten Testträger gemäß Figur 6 wird auf das Schutznetz (16) 32 »l Blut aufgegeben. Nach 60 Sekunden werden durch Druck auf die transparente Folie (11) das Enzympapier (12) und das Indikatorpapier (13) auf das Transportvlies (14) und das darin befindliche Serum gedrückt. Nach 120 Sekunden wird die Remission in Prozent bei 642 nm durch die transparente Folie (11) hindurch gemessen. Bei Einsatz bekannter aber verschiedener Ethanolkonzentrationen im Blut erhält man eine Kurve gemäß Figur 7. Diese Kurve kann als Eichkurve zur Bestimmung einer unbekannten Ethanolkonzentration in einer Probe dienen.

### Beispiel 6

### Bestimmung von β-Galactosidase (EC 3.2.1.23)

Folgende Lösungen werden angesetzt:
- Puffer:: 0,1 M HEPES, 20 mM Magnesiumchlorid, pH 6,8
- Substrat:: 50 mM p-Aminophenyl-β-Galactosid (hergestellt aus p-Nitrophenyl-β-D-galactosid durch Hydrierung mit Wasserstoff an Pd-Kohle gemäß "Organikum", VEB Deutscher Verlag der Wissenschaften, 15. Auflage, Berlin 1976) in Wasser
- Neutralisator:: 1 N Natronlauge
- Indikator:: 100 mg/ml 18 Molybdodiphosphorsäure und 50 mg/ml Phenyl-trimethyl-ammoniumchlorid in Wasser
- Enzym:: 180 U/ml in Puffer (die angegebene Enzymaktivität bezieht sich auf die Verwendung von o-Nitrophenol-galactosid als Substrat)

Zum Test werden in einem Reaktionsgemäß gemischt:

| | |
|---|---|
| Puffer | 780 »l |
| Indikator | 100 »l |
| Neutralisator | 20 »l |
| Substrat | 100 »l |
| Enzym | a) 0, b) 1 »l, c) 2 »l, d) 3 »l, e) 4 »l, f) 5 »l |

Nach 4 1/2 Minuten Inkubation wird für 1/2 Minute zentrifugiert, der Überstand verworfen, das Sediment in 1 ml Dimethylsulfoxid gelöst und sofort die Extinktion (E) gemessen. Es werden die Resultate der Tabelle 3 erhalten.

**Tabelle 3**

| »l Enzym | U/ml Enzym | E (800 nm) |
|---|---|---|
| 0 | 0 | 0,065 |
| 1 | 0,18 | 0,571 |
| 2 | 0,36 | 1,245 |
| 3 | 0,54 | 1,697 |
| 4 | 0,72 | 2,350 |
| 5 | 0,90 | 2,907 |

Die aus diesen Werten resultierende Eichkurve kann zur Ermittlung des β-Galactosidase-Gehalts in unbekannten Proben verwendet werden.

### Beispiel 7

### Bestimmung von alkalischer Phosphatase (EC 3.1.3.1)

Werden in der Rezeptur von Beispiel 6 als Substrat 50 mM p-Aminophenylphosphat (hergestellt gemäß L. H. De Riemer, C. F. Meares, Biochemistry 20, 1606 (1981) verwendet und als Puffer 1 M Diethanolamin, 1 mM Magnesiumchlorid, 0,1 mM Zinkchlorid, pH 9,8 verwendet, so kann damit die Bestimmung des Enzyms alkalische Phosphatase erfolgen. Mit bekannten Enzymkonzentrationen wird wie in Beispiel 6 ein linearer Zusammenhang zwischen Enzymkonzentration und Extinktion bei 800 nm gefunden.

### Beispiel 8

### Testträger zum Nachweis von γ-Glutamyltransferase (EC 2.3.2.2)

Analog Beispiel 1 wird ein Testträger hergestellt, jedoch mit folgenden Änderungen:
In der zweiten Reagenzschicht wird der Puffer (2 Gew.-% Keltrol F in 0,2 M Citrat-Puffer, pH 6,0) durch 2 Gew.-% Keltrol F in Wasser ersetzt, N,N-bis-(2-hydroxyethyl)-p-nitrosoanilin x HCl und Glucoseoxidase werden weggelassen. Zwischen Transferkleber (2) und zweiter Reagenzschicht (3) wird ein Substrat-getränktes Papier eingelegt, das folgendermaßen hergestellt wird:
Ein Teebeutelpapier (12 g/m²) wird mit einer Lösung von 250 mM Glycylglycin und 20 mM γ-L-Glutamyl-3-carboxyl-1,4-phenylendiamin (hergestellt gemäß EP-A-0 103 823) in 250 mM Trispuffer, pH 7,6 getränkt und 20 Minuten bei 50 °C getrocknet.

In 0,1 M Tris-Puffer mit 10 mg/ml Rinderserum Albumin, pH 7,5 wird eine Verdünnungsreihe von γ-Glutamyltransferase hergestellt. 10 »l dieser Lösung werden auf einen wie vorstehend beschriebenen Testträger aufgegeben. Nach einer Minute werden bei 37 °C die in Tabelle 4 wiedergegebenen Remissionswerte in Prozent bei einer Wellenlänge von 950 nm gemessen.

**Tabelle 4**

| Enzymkonzentration U/ml | Remission bei 950 nm in % |
|---|---|
| 0 | 59,0 |
| 0,245 | 57,0 |
| 0,471 | 55,5 |
| 1,31 | 52,7 |
| 2,77 | 49,7 |
| 4,93 | 45,8 |
| 7,39 | 42,2 |
| 9,85 | 39,0 |

Die so erhaltene Kurve kann zur Bestimmung des unbekannten γ-Glutamyltransferase-Gehalts in Flüssigkeiten genutzt werden.

### Beispiel 9

### Testträger zum Nachweis von saurer Phosphatase (EC 3.1.3.2)

Analog Beispiel 8 wird ein Testträger zum Nachweis von saurer Phosphatase erhalten, wenn das Teebeutelpapier mit 10 mM p-Aminophenylphosphat (hergestellt gemäß L. H. De Riemer, C. F. Meares, Biochemistry 20, 1606 (1981) in 0,1 M Citrat-Puffer, pH 5,0 getränkt wird.

## Patentansprüche

1. Verwendung eines schwer löslichen Salzes einer Heteropolysäure zur Bestimmung eines Analyts, dadurch gekennzeichnet, daß der Analyt ein elektronenreiches aromatisches Amin ist oder zusammen mit einer weiteren Substanz zu einem solchen führt.

2. Verfahren zur Bestimmung eines Analyts mittels Heteropolyblau-Bildung, dadurch gekennzeichnet, daß der Analyt mit einer Substanz umgesetzt wird, die zu einem aromatischen elektronenreichen Amin führt, das mit einem schwer löslichen Salz einer Heteropolysäure kontaktiert wird.

3. Verfahren gemäß Anspruch 2, dadurch gekennzeichnet, daß ein schwer lösliches Salz einer Heteropolysäure des Molybdäns, des Molybdäns und Wolframs, des Vanadiums und Molybdäns oder des Vanadiums und Molybdäns und Wolframs mit Phosphor, Arsen, Silicium oder Germanium als Heteroatom eingesetzt wird.

4. Verfahren gemäß einem der Ansprüche 2 und 3, dadurch gekennzeichnet, daß als schwer lösliches Salz einer Heteropolysäure ein solches eingesetzt wird, dessen Kation größer ist als das Ammoniumion.

5. Verfahren gemäß einem der Ansprüche 2 bis 4, dadurch gekennzeichnet, daß als schwer lösliches Salz einer Heteropolysäure ein solches mit einem Kation der allgemeinen Formel I
R¹R²R³R⁴X⁺ (I)
in der
R¹, R², R³, R⁴ gleich oder verschieden sind und einen Alkyl-, Aryl- oder Aralkylrest bedeuten, oder Wasserstoff, wenn nicht alle Reste gleich sind, oder
wobei zwei Reste zusammen einen Alkylenrest bilden, und X ein Phosphor- oder Stickstoffatom darstellt,
eingesetzt wird.

6. Verfahren gemäß einem der Ansprüche 2 bis 4, dadurch gekennzeichnet, daß als schwer lösliches Salz einer Heteropolysäure ein solches mit einem Kation aus der Gruppe der quarternierten Stickstoff-Heteroaromaten eingesetzt wird.

7. Verfahren zur Bestimmung eines elektronenreichen aromatischen Amins durch Heteropolyblau-Bildung, dadurch gekennzeichnet, daß eine Lösung des zu bestimmenden elektronenreichen aromatischen Amins mit einem schwer löslichen Salz einer Heteropolysäure kontaktiert wird.

8. Mittel zur Bestimmung eines Analyts durch Heteropolyblau-Bildung, dadurch gekennzeichnet, daß es eine Substanz, die mit dem Analyt zu einem elektronenreichen aromatischen Amin führt und ein schwer lösliches Salz einer Heteropolysäure oder die hierfür notwendigen Substanzen enthält.

9. Mittel gemäß Anspruch 8, dadurch gekennzeichnet, daß es ein schwer lösliches Salz einer Heteropolysäure des Molybdäns, des Molybdäns und Wolframs, des Vanadiums und Molybdäns oder des Vanadiums und Molybdäns und Wolframs mit Phosphor, Arsen, Silicium oder Germanium als Heteroatom enthält.

10. Mittel gemäß einem der Ansprüche 8 und 9, dadurch gekennzeichnet, daß es als schwer lösliches Salz einer Heteropolysäure ein solches enthält, dessen Kation größer ist als das Ammoniumion.

11. Mittel gemäß einem der Ansprüche 8 bis 10, dadurch gekennzeichnet, daß es als schwer lösliches Salz einer Heteropolysäure ein solches mit einem Kation der allgemeinen Formel I
R¹R²R³R⁴X⁺ (I)
in der
R¹, R², R³, R⁴ gleich oder verschieden sind und einen Alkyl-, Aryl- oder Aralkylrest bedeuten, oder Wasserstoff, wenn nicht alle Reste gleich sind, oder
wobei zwei Reste zusammen einen Alkylenrest bilden, und X ein Phosphor- oder Stickstoffatom darstellt,
enthält.

12. Mittel gemäß einem der Ansprüche 8 bis 10, dadurch gekennzeichnet, daß es als schwer lösliches Salz einer Heteropolysäure ein solches mit einem Kation aus der Gruppe der quaternierten Stickstoff-Heteroaromaten enthält.

13. Mittel zur Bestimmung eines elektronenreichen aromatischen Amins durch Heteropolyblau-Bildung, dadurch gekennzeichnet, daß es ein schwer lösliches Salz einer Heteropolysäure oder die hierfür notwendigen Substanzen in trägergebundener Form enthält.

14. Verwendung eines schwer löslichen Salzes einer Heteropolysäure zur Herstellung eines Mittels zur Bestimmung eines elektronenreichen aromatischen Amins durch Heteropolyblau-Bildung.

## Claims

1. Use of a sparingly soluble salt of a heteropoly acid for the determination of an analyte, characterised in that the analyte is an electron-rich aromatic amine or leads to such together with a further substance.

2. Process for the determination of an analyte by means of heteropoly blue formation, characterised in that the analyte is reacted with a substance which leads to an aromatic electron-rich amine which is contacted with a sparingly soluble salt of a heteropoly acid.

3. Process according to claim 2, characterised in that a sparingly soluble salt of a heteropoly acid of molybdenum, of molybdenum and tungsten, of vanadium and molybdenum or of vanadium and molybdenum and tungsten, with phosphorus, arsenic, silicon or germanium as heteroatom, is used.

4. Process according to one of claims 2 and 3, characterised in that, as sparingly soluble salt of a heteropoly acid, such a one is used, the cation of which is larger than the ammonium ion.

5. Process according to one of claims 2 to 4, characterised in that, as sparingly soluble salt of a heteropoly acid, such a one is used with a cation of the general formula I
R¹R²R³R⁴X⁺ (I)
in which R¹, R², R³, R⁴ are the same or different and signify an alkyl, aryl or aralkyl radical or hydrogen when not all radicals are the same or whereby two radicals together form an alkylene radical and X represents a phosphorus or nitrogen atom.

6. Process according to one of claims 2 to 4, characterised in that, as sparingly soluble salt of a heteropoly acid, such a one is used with a cation from the group of the quaternised nitrogen heteroaromatics.

7. Process for the determination of an electronrich aromatic amine by heteropoly blue formation, characterised in that a solution of the electron-rich aromatic amine to be determined is contacted with a sparingly soluble salt of a heteropoly acid.

8. Agent for the determination of an analyte by heteropoly blue formation, characterised in that it contains a substance which, with the analyte, leads to an electron-rich aromatic amine and contains a sparingly soluble salt of a heteropoly acid or the substances necessary herefor.

9. Agent according to claim 8, characterised in that it contains a sparingly soluble salt of a heteropoly acid of molybdenum, of molybdenum and tungsten, of vanadium and molybdenum or of vanadium and molybdenum and tungsten with phosphorus, arsenic, silicon or germanium as heteroatom.

10. Agent according to claims 8 and 9, characterised in that, as sparingly soluble salt of a heteropoly acid, it contains such a one, the cation of which is larger than the ammonium ion.

11. Agent according to one of claims 8 to 10, characterised in that, as sparingly soluble salt of a heteropoly acid, it contains such a one with a cation of the general formula I
R¹R²R³R⁴X⁺ (I)
in which R¹, R², R³, R⁴ are the same or different and signify an alkyl, aryl or aralkyl radical or hydrogen when not all radicals are the same or whereby two radicals together form an alkylene radical and X represents a phosphorus or nitrogen atom.

12. Agent according to one of claims 8 to 10, characterised in that, as sparingly soluble salt of a heteropoly acid, it contains such a one with a cation from the group of the quaternised nitrogen heteroaromatics.

13. Agent for the determination of an electron-rich aromatic amine by heteropoly blue formation, characterised in that it contains a sparingly soluble salt of a heteropoly acid or the substances necessary herefor in carrier-bound form.

14. Use of a sparingly soluble salt of a heteropoly acid for the production of an agent for the determination of an electron-rich aromatic amine by heteropoly blue formation.

## Revendications

1. Utilisation d'un sel peu soluble d'un hétéropolyacide pour la détermination d'un analyte, caractérisée en ce que l'analyte est une amine aromatique riche en électrons, ou conduit à celle-ci conjointement avec une autre substance.

2. Procédé de détermination d'un analyte par formation d'hétéropoly-bleu, caractérisé en ce que l'on fait réagir l'analyte avec une substance qui conduit à une amine aromatique riche en électrons, que l'on met en contact avec un sel peu soluble d'un hétéropolyacide.

3. Procédé selon la revendication 2, caractérisé en ce que l'on utilise un sel peu soluble d'un hétéropolyacide du molybdène, du molybdène et du tungstène, du vanadium et du molybdène ou du vanadium et du molybdène et du tungstène, avec du phosphore, de l'arsenic, du silicium ou du germanium en tant qu'hétéroatome.

4. Procédé selon l'une des revendications 2 et 3, caractérisé en ce que comme sel peu soluble d'un hétéropolyacide, on utilise un sel dont le cation présente une taille supérieure à celle de l'ion ammonium.

5. Procédé selon l'une quelconque des revendications 2 à 4, caractérisé en ce que comme sel peu soluble d'un hétéropolyacide, on utilise un sel contenant un cation de formule générale I
R¹R²R³R⁴X⁺ (I)
dans laquelle
R¹, R², R³, R⁴ sont identiques ou différents et représentent un reste alkyle, aryle ou aralkyle, ou un atome d'hydrogène lorsque tous les restes ne sont pas identiques, ou
dans laquelle deux restes forment conjointement un reste alcényle, et X représente un atome de phosphore ou d'azote.

6. Procédé selon l'une quelconque des revendications 2 à 4, caractérisé en ce que comme sel peu soluble d'un hétéropolyacide, on utilise un sel contenant un cation appartenant au groupe des composés hétéroaromatiques à azote quaternaire.

7. Procédé de détermination d'une amine aromatique riche en électrons, par formation d'hétéropoly-bleu, caractérisé en ce que l'on met en contact une solution de l'amine aromatique riche en électrons à déterminer avec un sel peu soluble d'un hétéropolyacide.

8. Agent de détermination d'un analyte par formation d'hétéropoly-bleu, caractérisé en ce qu'il contient une substance qui conduit, avec l'analyte, à une amine aromatique riche en électrons et qui contient un sel peu soluble d'un hétéropolyacide ou les substances nécessaires à son obtention.

9. Agent selon la revendication 8, caractérisé en ce qu'il contient un sel peu soluble d'un hétéropolyacide du molybdène, du molybdène et du tungstène, du vanadium et du molybdène ou du vanadium et du molybdène et du tungstène, avec du phosphore, de l'arsenic, du silicium ou du germanium en tant qu'hétéroatome.

10. Agent selon l'une des revendications 8 et 9, caractérisé en ce que comme sel peu soluble d'un hétéropolyacide, il contient un sel dont le cation présente une taille supérieure à celle de l'ion ammonium.

11. Agent selon l'une quelconque des revendications 8 à 10, caractérisé en ce que, comme sel peu soluble d'un hétéropolyacide, il comprend un sel contenant un cation de formule générale I
R¹R²R³R⁴X⁺ (I)
dans laquelle
R¹, R², R³, R⁴ sont identiques ou différents et représentent un reste alkyle, aryle ou aralkyle, ou un atome d'hydrogène lorsque tous les restes ne sont pas identiques, ou
dans laquelle deux restes forment conjointement un reste alcényle, et X représente un atome de phosphore ou d'azote.

12. Agent selon l'une quelconque des revendications 8 à 10, caractérisé en ce que, comme sel peu soluble d'un hétéropolyacide, il comprend un sel contenant un cation appartenant au groupe des composés hétéroaromatiques à note quaternaire.

13. Agent pour la détermination d'une amine aromatique riche en électrons, par formation d'hétéropoly-bleu, caractérisé en ce qu'il contient un sel peu soluble d'un hétéropolyacide, ou les substances nécessaires à son obtention, sous forme lié à un support.

14. Utilisation d'un sel peu soluble d'un hétéropolyacide pour la préparation d'un agent pour la détermination d'une amine aromatique riche en électrons, par formation d'hétéropoly-bleu.
